# EUROPEAN PATENT SPECIFICATION

(11) **EP 2 148 655 B1**
(45) Date of publication and mention of the grant of the patent: **27.02.2013**
(21) Application number: 08737878.2
(22) Date of filing: 16.04.2008
(51) Int. Cl.: A61K 9/16, A61K 9/20, A61K 31/4365, A61K 9/26, A61K 9/28, A61K 9/32, A61K 9/36

(54) **PHARMACEUTICAL COMPOSITIONS OF CLOPIDOGREL**
PHARMAZEUTISCHE ZUSAMMENSETZUNGEN VON CLOPIDOGREL
COMPOSITIONS PHARMACEUTIQUES DE CLOPIDOGREL

(30) Priority: 20.04.2007 IN MU07642007; 20.04.2007 IN MU07742007
(43) Date of publication of application: 03.02.2010
(73) Proprietor: Wockhardt Limited, Aurangabad 431210 (IN); Ujjainkar, Amol, Aurangabad (IN); Balla Pattabhi, Srinivas, Vizianagaram 535002 (IN); Khan, Abdul Rehman, Aurangabad 431 003 MAH (IN); Murali, Narayanan, Korattur Chennai 600080 (IN); Jain, Girish Kumar, New Delhi 110 034 (IN)
(72) Inventor: UJJAINKAR, Amol, Aurangabad (IN); BALLA PATTABHI, Srinivas, Vizianagaram 535002 (IN); KHAN, Abdul Rehman, Aurangabad 431003 (IN); MURALI, Narayanan, Chennai 600080 (IN); JAIN, Girish Kumar, Delhi 110034 (IN)
(74) Representative: May, Mark Andrew
(86) International application number: PCT/IB2008/051456
(87) International publication number: WO 2008/129468

(56) References cited:
- WO-A-2004/074215
- WO-A-2004/098593
- WO-A-2005/048992
- WO-A-2006/074066
- WO-A-2007/091279
- WO-A-2008/001201
- US-A1- 2007 048 370
- US-B1- 6 218 403

## Description

Field of the Invention

The invention relates to pharmaceutical compositions comprising clopidogrel or monobasic acid salts thereof in admixture with one or more hydrated excipients. The invention also relates to pharmaceutical compositions comprising clopidogrel or monobasic acid salts thereof which are free of any moisture scavenger. The invention also relates to processes for the preparation of such compositions.

Background of the Invention

Clopidogrel is an inhibitor of ADP-induced platelet aggregation acting by direct inhibition of adenosine diphosphate (ADP) binding to its receptor and of the subsequent ADP-mediated activation of the glycoprotein GPIIb/IIIa complex. Chemically, it is (α S)-α- (2-chlorophenyl) -6,7-dihydrothieno [3,2-c] pyridine-5 (4H)-acetic acid methyl ester. It is indicated for the reduction of atherothrombotic events in patients with history of recent myocardial infarction (MI), recent stroke, or established peripheral arterial disease and acute coronary syndrome.

U.S. Patent Application No. 20070048370 discloses clopidogrel tablet formulation, wherein the tablet contains no ionic and/or basic tabletting excipients and no PEG 6000.

International (PCT) Publication No. WO2005048992 discloses compositions of clopidogrel, wherein the clopidogrel is in the form of coated particles mixed with anhydrous excipients along with moisture scavengers.

International (PCT) Publication Nos. WO2005117866 and WO2006074066 disclose amorphous form of clopidogrel hydrochloride and its compositions.

International (PCT) Publication No. WO2007091279 discloses a pharmaceutical composition of clopidogrel bisulphate and a lubricant glyceryldibehenate.

International (PCT) Publication No. WO20 08001201 discloses a melt-granulated pharmaceutical composition of clopidogrel or salts thereof.

Clopidogrel in a base or a monobasic acid salt form, such as mesylate, hydrochloride, hydrobromide, benzoate, salicylate, lactate, gluconate is known to exhibit difficulties in formulating solid stable dosage forms, owing to its hygroscopicity. The monobasic acid salts of clopidogrel are known to interact with certain formulation excipients and undergo degradation. The pharmaceutical compositions containing clopidogrel or monobasic acid salts thereof are not commercially available. To overcome such problems, usually the bisulphate salt of clopidogrel is used.

During development of pharmaceutical formulations comprising clopidogrel hydrochloride, often drug tends to degrade even in the presence of a little moisture and thus makes the drug unavailable in the plasma at the time of ingestion. Several references describe formulations, wherein the clopidogrel is either in the form of coated particles or mixed with anhydrous excipients along with moisture scavengers. Clearly, there is a need for improved compositions which are not only easy to prepare and does not utilize anhydrous excipients along with moisture scavengers but are also bioequivalent to the commercially available clopidogrel bisulphate tablets (Plavix^{®}).

### Summary of the Invention

In one general aspect there is provided a pharmaceutical composition that includes clopidogrel or monobasic acid salts thereof, and one or more hydrated excipients.

The term 'hydrated excipients' as used herein refers to sufficient amount of water present in them so as to get them in the hydrated state.

Embodiments of the pharmaceutical composition may include one or more of the following features. The pharmaceutical composition may further include one or more pharmaceutically acceptable excipients. The pharmaceutically acceptable excipients may include one or more of fillers, binders, lubricants, glidants, disintegrants, and the like.

In another general aspect there is provided a pharmaceutical composition that includes uncoated granules, particles or pellets of clopidogrel or monobasic acid salts thereof, wherein the composition is free of any moisture scavenger.

The term 'moisture scavenger' as used herein refers to a substance which scavenges free moisture and prevents degradation or decomposition or sticking or hygroscopicity of the active ingredient.

The term 'uncoated granules, particles or pellets of clopidogrel' as used herein refers to granules, particles or pellets of clopidogrel which are as such and not coated with any polymer.

Embodiments of the pharmaceutical composition may include one or more of the following features. The pharmaceutical composition may further include one or more pharmaceutically acceptable excipients. The pharmaceutically acceptable excipients may include one or more of fillers, binders, lubricants, glidants, disintegrants, and the like.

In another general aspect there is provided a pharmaceutical composition that includes clopidogrel hydrochloride and glyceryl behenate, wherein one or both of the rate and the extent of absorption of the clopidogrel hydrochloride is equal to or greater than that obtained by a clopidogrel bisulphate tablet marketed under the trade name Plavix^{®}.

In another general aspect there are provided processes for preparing pharmaceutical compositions that include clopidogrel or monobasic acid salts thereof. The process includes: a) mixing and granulating clopidogrel or monobasic acid salts thereof with hydrated excipients; and b) converting the granules into a suitable dosage form.

Embodiments of the pharmaceutical composition may include one or more of the following features. The pharmaceutical composition may further include one or more pharmaceutically acceptable excipients. The pharmaceutically acceptable excipients may include one or more of fillers, binders, lubricants, glidants, disintegrants, and the like.

The details of one or more embodiments of the inventions are set forth in the description below. Other features, objects and advantages of the inventions will be apparent from the description and claims.

Description of drawings

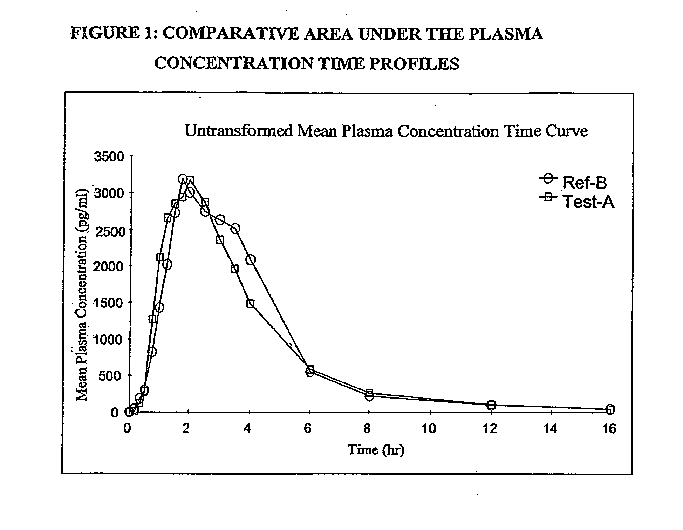

Figure 1: Comparative in vivo area under the plasma concentration time profiles (AUC)

Detailed Description of the Invention

We have now discovered that clopidogrel or monobasic acid salts thereof, when mixed with hydrated excipients results in a composition which is stable that does not undergo degradation on storage. Further, we have discovered that a stable formulation of clopidogrel or monobasic acids salts thereof can be prepared without coating with any hydrophobic polymer. It was also found that a stable formulation can be prepared when the formulation does not contain any moisture scavenger. The inventors have surprisingly found that a stable formulation of clopidogrel or monobasic acid salts thereof can be prepared using glyceryl behenate in the formulation, which is bioequivalent to clopidogrel bisulfate tablet dosage form commercially marketed under the trade name Plavix^{®}.

The monobasic acid salts of clopidogrel can be selected from one or more of clopidogrel hydrochloride, clopidogrel hydrobromide, clopidogrel mesylate, clopidogrel besylate, clopidogrel salicylate, clopidogrel lactate or clopidogrel gluconate.

The hydrated excipients can be selected from one or more of microcrystalline cellulose such as Avicel PH 101, low substituted hydroxy propyl cellulose, high substituted hydroxypropyl cellulose, hydroxypropyl methyl cellulose, colloidal silicon dioxide and the like.

In general, the pharmaceutical compositions of the invention can be prepared by simple mixing, direct compression, dry granulation or wet granulation.

In one embodiment the pharmaceutical composition of clopidogrel or monobasic acid salts thereof may be prepared by compacting clopidogrel or monobasic acid salts there of with pharmaceutically acceptable excipients optionally with lubricant. The aggregates thus obtained may be sized into granules or particles, which may be mixed with other pharmaceutically acceptable excipients, may be formulated in suitable dosage form.

The pharmaceutical compositions may include one or more pharmaceutically acceptable excipients selected from fillers, binders, lubricants, glidants, disintegrants, and the like.

Suitable fillers may include one or more of lactose, microcrystalline cellulose, polyethylene glycols, mannitol, calcium phosphate, calcium sulfate, kaolin, dry starch, sorbitol, powdered sugar, prosolv, and the like.

Suitable disintegrants may include one or more of starch, croscarmellose sodium, crospovidone, sodium starch glycolate, hydroxypropylcellulose, and the like.

Suitable glidants may include one or more of colloidal silicon dioxide, talc or cornstarch, and the like.

Suitable lubricants comprises one or more of hydrogenated vegetable oil, hydrogenated castor oil, light mineral oil, glycerol monostearate, glycerol monobehenate, glyceryl behenate, glyceryl palmitostearate, and the like.

The pharmaceutical composition of the invention can be present in the form of granules, pellets, beads, spheroids, a tablet, a minitablet, a microtablet, a capsule, granules in a capsule, pellets in a capsule, minitablets in a capsule, or combinations thereof.

'Bioequivalence' is established by a 90% Confidence Interval (CI) of between 0.80 and 1.25 for both Cₘₐₓ and AUC under USFDA regulatory guidelines, or a 90% CI for AUC of between 0.80 to 1.25 and a 90% CI for Cₘₐₓ of between 0.70 to 1.43 under the European EMEA regulatory guidelines.

Bioequivalence studies were carried out between Plavix® and composition of the invention both in fed state and fasted state. The study was monitored in terms of Cmax, AUC, Tmax achieved with the test product and reference product (Plavix®). Table 1 gives bioequivalence data of Plavix® and test product.

Each subject received a single dose of clopidogrel after starting breakfast. Wash out periods of one week separated doses.

Blood samples were collected prior to dosing (0 hour) and at 0.16, 0.33, 0.5, 0.75, 1.0, 1.25, 1.5, 1.75, 2.0, 2.5, 3.0, 3.5, 4.0, 6.0, 8.0, 12, and 16 hour after each dose. Plasma samples were assayed for clopidogrel using a validated high performance liquid chromatographic procedure. Values for clopidogrel pharmacokinetic parameters including observed Cₘₐₓ. AUC₀₋ₜ and AUC_{0-∞}, were calculated using standard methods.

Table 1: Bioequivalence data with respect to Test (Composition of the invention) to reference (Plavix®) Ratios (T/R ratios) at 90% Confidence Interval (C.I.)

[Table 1]

**[Table]**

| **Pharmacokinetic parameters** | **Ratio % (Test/Ref)** | |
|---|---|---|
| Cmax | 1. | 81 |
| AUC₀₋ₜ | 1. | 35 |
| AUC_{0-∞} | 1. | 97 |

The invention is further illustrated by the following examples which are provided merely to be exemplary of the invention and do not limit the scope of the invention. Certain modifications and equivalents will be apparent to those skilled in the art and are intended to be included within the scope of the invention.

Example 1 : The composition of batches is provided in Table 1.

Table-1:

[Table 2]

**[Table]**

| **S.No** | **Ingredients** | **Quantity/tablet (% w/w)** |
|---|---|---|
| 1. | Clopidogrel hydrochloride | 15-35 |
| 2. | Microcrystalline cellulose | 30-70 |
| 3. | Low substituted hydroxy propyl cellulose (L-HPC) | 0.1-20 |
| 4. | Colloidal silicon dioxide | 0.5-5 |
| 5. | Glyceryl behenate | 0.1-5 |
| 6. | Hydrogenated castor oil | 0.5-5 |
| 7. | Opadry | 0.5-5.0 |

Procedure: Clopidogrel hydrochloride was mixed with microcrystalline cellulose, low substituted hydroxypropyl cellulose, colloidal silicon dioxide and glyceryl behenate in a double cone blender. The blend obtained was compacted using a roll compactor. The aggregates were sized into granules using oscillating granulator. The granules were mixed with low substituted hydroxypropyl cellulose, colloidal silicon dioxide, crospovidone, talc and lubricated with glyceryl behenate, hydrogenated castor oil in a double cone blender. The lubricated blend was finally compressed into tablets using a suitable tooling. The tablets were coated with aqueous dispersion of Opadry.

Example-2 : The composition of the batches is provided in Table 2.

Table-2:

[Table 3]

**[Table]**

| **S.No** | **Ingredients** | **%w/w** |
|---|---|---|
| | **Intragranular** | |
| 1 | Clopidogrel Hydrochloride | 15-40 |
| 2 | Silicified microcrystalline cellulose | 30-70 |
| 3 | Low substituted HPC | 5-10 |
| 4 | Glyceryl Behenate | 1-5 |

| | **Extragranular** | |
|---|---|---|
| 5 | Low substituted HPC (LH -11) | 0.5-2.0 |
| 6 | Silicified microcrystalline cellulose | 5-10 |
| 7 | Glyceryl Behenate | 1-5 |
| 8 | Hydrogenated Castor oil | 1-5 |

Procedure: Clopidogrel hydrochloride was mixed with Prosolv, low substituted hydroxypropyl cellulose, and glyceryl behenate in a double cone blender and the blend was compacted using a roller compactor. The aggregates were sized into granules using oscillating granulator. The granules were mixed with Prosolv, low substituted hydroxypropyl cellulose and lubricated with glyceryl behenate, hydrogenated castor oil in a double cone blender. The lubricated blend was finally compressed into tablets using a suitable tooling. The tablets were coated with aqueous dispersion of Opadry.

## Claims

1. A pharmaceutical composition comprising a non-melt granulated form of clopidogrel or monobasic acid salts thereof and one or more hydrated excipients selected from the group consisting of microcrystalline cellulose low substituted hydroxy propyl cellulose, high substituted hydroxy propyl cellulose, hydroxy propyl methyl cellulose and colloidal silicon dioxide, and glyceryl behenate, wherein the composition is free of any moisture scavenger and of anhydrous lactose.

2. The pharmaceutical composition according to claim 1, wherein the composition comprisies uncoated granules, particles or pellets of clopidogrel or monobasic acid salts thereof.

3. The pharmaceutical composition according to claim 1 or 2, wherein the monobasic acid salt of clopidogrel comprises one or more of clopidogrel hydrochloride, clopidogrel hydrobromide, clopidogrel mesylate, clopidogrel besylate, clopidogrel salicylate, clopidogrel lactate and clopidogrel gluconate.

4. The pharmaceutical composition according to any of claims 1 to 3, further comprising one or more pharmaceutically acceptable excipients comprising filler, binder, lubricant, glidant and disintegrant.

5. The pharmaceutical composition according to claim 4, wherein the lubricant comprises one or more of hydrogenated vegetable oil, hydrogenated castor oil, light mineral oil, glycerol monostearate, glycerol monobehenate, glyceryl behenate, and glyceryl palmitostearate.

6. The pharmaceutical composition according to any of the claims 1 to 3, wherein the composition comprises one or more of granules, pellets, beads, spheroids, a tablet; a minitablet, a microtablet, a capsule, granules in a capsule, pellets in a capsule, macrotablets in a capsule and minitablets in a capsule.

7. A process for preparing a pharmaceutical composition comprising clopidogrel or monobasic acid salts thereof according to claim 1, comprising (a) mixing and granulating clopidogrel or monobasic acid salts thereof with one or more of hydrated excipients selected from the group consisting of microcrystalline cellulose low substituted hydroxy propyl cellulose, high substituted hydroxy propyl cellulose, hydroxy propyl methyl cellulose and colloidal silicon dioxide; and (b) converting the granules into a suitable dosage form.

8. The process according to claim 7, wherein the monobasic acid salt of clopidogrel comprises one or more of clopidogrel hydrochloride, clopidogrel hydrobromide, clopidogrel mesylate, clopidogrel besylate, clopidogrel salicylate, clopidogrel lactate and clopidogrel gluconate.

9. The process according to claim 7, further comprising one or more pharmaceutically acceptable excipients comprising filler, binder, lubricant, glidant and disintegrant

10. The process according to claim 7, wherein the lubricant comprises one or more of hydrogenated vegetable oil, hydrogenated castor oil, light mineral oiL glycerol monostearate, glycerol monobehenate, glyceryl behenate, and glyceryl palmitostearate.

11. The process according to claim 7, wherein the composition comprises one or more of granules, pellets, beads, spheroids, a tablet, a minitablet, a microtablet, a capsule, granules in a capsule, pellets in a capsule, macrotablets in a capsule and minitablets in a capsule.

## Patentansprüche

1. Pharmazeutische Zusammensetzung, umfassend eine nicht schmelzgranulierte Form von Clopidogrel oder eines monobasischen Salzes desselben und einen oder mehrere hydratisierte Hilfsstoffe, ausgewählt aus der Gruppe bestehend aus mikrokristalliner Cellulose, niedrig substituierter Hydroxypropylcellulose, hoch substituierter Hydroxypropylcellulose, Hydroxypropylmethylcellulose und kolloidalem Siliziumdioxid, und Glycerylbehenat, wobei die Zusammensetzung frei von Feuchtigkeitsadsorbern und von wasserfreier Lactose ist.

2. Pharmazeutische Zusammensetzung nach Anspruch 1, wobei die Zusammensetzung unbeschichtete Granalien, Partikel oder Pellets von Clopidogrel oder eines monobasischen Salzes desselben umfasst.

3. Pharmazeutische Zusammensetzung nach Anspruch 1 oder 2, wobei das monobasische Salz von Clopidogrel ein oder mehrere von Clopidogrelhydrochlorid, Clopidogrelhydrobromid, Clopidogrelmesylat, Clopidogrelbesylat, Clopidogrelsalicylat, Clopidogrellactat und Clopidogrelgluconat umfasst.

4. Pharmazeutische Zusammensetzung nach einem der Ansprüche 1 bis 3, ferner einen oder mehrere pharmazeutisch verträgliche Hilfsstoffe, umfassend Füllstoffe, Bindemittel, Gleitmittel, Flussregulierungsmittel und Sprengmittel, enthaltend.

5. Pharmazeutische Zusammensetzung nach Anspruch 4, wobei das Gleitmittel eins oder mehrere von hydriertem Pflanzenöl, hydriertem Rhizinusöl, leichtem Mineralöl, Glycerolmonostearat, Glycerolmonobehenat und Glycerylpalmitostearat umfasst.

6. Pharmazeutische Zusammensetzung nach einem der Ansprüche 1 bis 3, wobei die Zusammensetzung ein oder mehrere von Granulat, Pellets, Kügelchen, Sphäroide, eine Tablette, eine Minitablette, eine Mikrotablette, eine Kapsel, Granulat in einer Kapsel, Pellets in einer Kapsel, Makrotabletten in einer Kapsel und Minitabletten in einer Kapsel umfasst.

7. Verfahren zum Herstellen einer pharmazeutischen Zusammensetzung, umfassend (a) mischen und granulieren von Clopidogrel oder eines monobasischen Salzes desselben mit einem oder mehreren hydratisierten Hilfsstoffen, ausgewählt aus der Gruppe bestehend aus mikrokristalliner Cellulose, niedrig substituierter Hydroxypropylcellulose, hoch substituierter Hydroxypropylcellulose, Hydroxypropylmethylcellulose und kolloidalem Siliziumdioxid; und (b) konvertieren des Granulats in eine geeignete Darreichungsform.

8. Verfahren nach Anspruch 7, in dem das monobasische Salz von Clopidogrel ein oder mehrere von Clopidogrelhydrochlorid, Clopidogrelhydrobromid, Clopidogrelmesylat, Clopidogrelbesylat, Clopidogrelsalicylat, Clopidogrellactat und Clopidogrelgluconat umfasst.

9. Verfahren nach Anspruch 7, ferner beinhaltend einen oder mehrere pharmazeutisch verträgliche Hilfsstoffe, umfassend Füllstoffe, Bindemittel, Gleitmittel, Flussregulierungsmittel und Sprengmittel.

10. Verfahren nach Anspruch 7, bei dem das Gleitmittel eins oder mehrere von hydriertem Pflanzenöl, hydriertem Rhizinusöl, leichtem Mineralöl, Glycerolmonostearat, Glycerolmonobehenat und Glycerylpalmitostearat umfasst.

11. Verfahren nach Anspruch7, bei dem die Zusammensetzung ein oder mehrere von Granulat, Pellets, Kügelchen, Sphäroide, eine Tablette, eine Minitablette, eine Mikrotablette, eine Kapsel, Granulat in einer Kapsel, Pellets in einer Kapsel, Makrotabletten in einer Kapsel und Minitabletten in einer Kapsel umfasst.

## Revendications

1. Composition pharmaceutique comprenant une forme non granulée à l'état fondu du clopidogrel ou de ses sels d'acide monobasique et un ou plusieurs excipients hydratés choisis dans le groupe comprenant de la cellulose microcristalline, de l'hydroxy propyl cellulose à faible substitution, de l'hydroxy propyl cellulose à substitution élevée, de l'hydroxy propyl méthyl cellulose et du dioxyde de silicium colloïdal, et du béhénate de glycéryle, la composition étant exempte de tout agent de fixation de l'humidité et de lactose anhydre.

2. Composition pharmaceutique selon la revendication 1, dans laquelle la composition comprend des granulés, des particules ou des pastilles non enrobés de clopidogrel ou de ses sels d'acide monobasique.

3. Composition pharmaceutique selon la revendication 1 ou 2, dans laquelle le sel d'acide monobasique du clopidogrel comprend un ou plusieurs composés parmi le chlorhydrate de clopidogrel, le bromhydrate de clopidogrel, le mésylate de clopidogrel, le bésylate de clopidogrel, le salicylate de clopidogrel, le lactate de clopidogrel et le gluconate de clopidogrel.

4. Composition pharmaceutique selon l'une quelconque des revendications 1 à 3, comprenant en outre un ou plusieurs excipients pharmaceutiquement acceptables comprenant une charge, un liant, un lubrifiant, un agent de glissement et un délitant.

5. Composition pharmaceutique selon la revendication 4, dans laquelle le lubrifiant comprend un ou plusieurs composés parmi une huile végétale hydrogénée, de l'huile de ricin hydrogénée, une huile minérale légère, le monostéarate de glycérol, le monobéhénate de glycérol, le béhénate de glycéryle, et le palmitostéarate de glycéryle.

6. Composition pharmaceutique selon l'une quelconque des revendications 1 à 3, dans laquelle la composition comprend un ou plusieurs éléments parmi des granulés, des pastilles, des billes, des sphéroïdes, un comprimé, un minicomprimé, un microcomprimé, une capsule, des granulés dans une capsule, des pastilles dans une capsule, des macrocomprimés dans une capsule et des minicomprimés dans une capsule.

7. Procédé de préparation d'une composition pharmaceutique comprenant du clopidogrel ou des sels d'acide monobasique de celui-ci selon la revendication 1, comprenant les étapes consistant à (a) mélanger et former des granulés de clopidogrel ou de ses sels d'acide monobasique avec un ou plusieurs excipients hydratés choisis dans le groupe comprenant de la cellulose microcristalline, de l'hydroxy propyl cellulose à faible substitution, de l'hydroxy propyl cellulose à substitution élevée, de l'hydroxy propyl méthyl cellulose et du dioxyde de silicium colloïdal ;
et à (b) convertir les granulés en une forme galénique appropriée.

8. Procédé selon la revendication 7, dans lequel le sel d'acide monobasique du clopidogrel comprend un ou plusieurs composés parmi le chlorhydrate de clopidogrel, le bromhydrate de clopidogrel, le mésylate de clopidogrel, le bésylate de clopidogrel, le salicylate de clopidogrel, le lactate de clopidogrel et le gluconate de clopidogrel.

9. Procédé selon la revendication 7, comprenant en outre un ou plusieurs excipients pharmaceutiquement acceptables comprenant une charge, un liant, un lubrifiant, un agent de glissement et un délitant.

10. Procédé selon la revendication 7, dans lequel le lubrifiant comprend un ou plusieurs composés parmi une huile végétale hydrogénée, de l'huile de ricin hydrogénée, une huile minérale légère, le monostéarate de glycérol, le monobéhénate de glycérol, le béhénate de glycéryle, et le palmitostéarate de glycéryle.

11. Procédé selon la revendication 7, dans lequel 1a composition comprend un ou plusieurs éléments parmi des granulés, des pastilles, des billes, des sphéroïdes, un comprimé, un minicomprimé, un microcomprimé, une capsule, des granulés dans une capsule, des pastilles dans une capsule, des macrocomprimés dans une capsule et des minicomprimés dans une capsule.
